# EUROPEAN PATENT SPECIFICATION

(11) **EP 1 766 386 B1**
(45) Date of publication and mention of the grant of the patent: **15.05.2013**
(21) Application number: 04776538.3
(22) Date of filing: 14.06.2004
(51) Int. Cl.: G01N 33/48, G01N 31/22, G01N 33/02, G01N 33/15

(54) **Product shelf life monitoring systems and ultraviolet radiation-absorbent ink.**
Systeme zur Überwachung der Produkthaltbarkeit und Tinte zum Absorbieren ultravioletter Strahlung.
Systèmes de gestion de la durée de conservation de produits et encre d'absorption de rayons ultraviolet.

(43) Date of publication of application: 28.03.2007
(73) Proprietor: Temptime Corporation, Morris Plains, New Jersey 07950 (US)
(72) Inventor: THADDEUS, Prusik, c/oTemptime Corporation, Morris Plains, NJ 07950 (US); PIECHOWSKI, Allan P., c/o Temptime Corporation, Morris Plains, NJ 07950 (US)
(74) Representative: Jorio, Paolo
(86) International application number: PCT/US2004/018847
(87) International publication number: WO 2006/001801

(56) References cited:
- EP-A- 1 048 477
- WO-A-99/53311
- DE-A1- 19 803 208
- JP-A- 4 067 804
- JP-A- 2001 049 190
- JP-A- 2003 313 499
- US-A- 4 789 637
- US-A1- 2004 253 733
- US-B1- 6 649 058

## Description

### BACKGROUND OF THE INVENTION

The present invention relates to a product shelf life monitoring system and to an ultraviolet radiation absorbent ink. The system, which may often take the form of a label, is associated with a product of foodstuff, medicament, or the like the useful shelf life, i.e., the safe or potent utility, of which is known to expire substantially concurrently with the elapse of the given time-temperature integral.

Substituted diacetylenic monomers useful in the present invention have been studied and utilized in TTI systems for many years. Such utility of numerous monomers comprising at least two conjugated acetylene groups (-C≡C-C≡C-) and their unique physico-chemical properties, e.g., responsiveness to temperature change by transforming into contrastingly colored solid state polymerization reaction products, have, for instance, been described by Patel et al. U.S.Patent No.3,999,946. The synthesis of these monomers and their implementation in useful TTI and other shelf life indicator compositions are discussed there at length by Patel et al. Likewise, the use of these diacetylenic monomer shelf life system components and improvements thereon, including asymmetric substitutions and complexes, and improved methods of monomer synthesis, have been described at length by Patel (U.S. 4,384,980) and Preziosi et al. (U.S. 4,788,151). Such diacetylenic monomer components are employed in the present invention.

While the consistent and predictable response of these diacetylenic monomer compositions to thermal stimuli provides a basis for highly functional and reliable TTI system products, a similar contemporary color-generating solid state polymerization response to other ambient actinic stimuli, such as ultraviolet radiation, significantly compromises their utility in such systems. This detraction has been recognized long since even by Patel et al who suggested the use of active ultraviolet light-absorbing compounds, such as benzophenones, benzotriazoles, and the like to mitigate these results. Thus, diacetylenic monomer composition TTI products comprising sheet elements, such as labels or marker tabs, bearing a localized deposit of active monomer indicator composition have had laminated thereto an overlying film comprising a UV-absorbing composition. By way of example, EP 1048477 teaches protecting acetylenic compounds from exposure with a UV-absorbing film over said compound. While such a fabrication practice has provided a TTI product with significantly reduced sensitivity to UV stimuli, the expanse of UV-blocking film usually far exceeds that needed to cover the relatively small deposit of active TTI composition component. As a result, a significant amount of expensive UV-absorber composition utilized in the fabrication process serves no advantageous purpose and merely represents a wasted cost factor resource. Further, in some TTI product applications the complete expanse of UV-blocking material prevents the selective employment of otherwise useful areas of a TTI label.

In particular, the present invention relates to responsive compositions of shelf life systems comprising substituted diacetylenic monomer components which exhibit a distinct color change as a result of and generally concomitant with a solid state polymerization effected by the ambient condition integral; typically, as in the case of a time-temperature indicator (TTI) system, the integral of time and temperature. More particularly, through incorporation of integral elements for isolating actinic stimuli. In some embodiments, the invention provides TTI systems and compositions of remarkable durability, sensitivity, and responsiveness which yield significant functional and economic improvements while greatly reducing the consumption of highly priced components and resources.

In some useful embodiments, the present invention obviates the noted shortcomings and disadvantages of prior diacetylenic monomer composition TTI system products and provides such products which yield effective results while reducing costs and achieving savings in fabrication time and material resources.

### SUMMARY OF THE INVENTION

The present invention provides a product shelf life monitoring system as defined in claim 1 and UV-radiation-absorbent ink as defined in claim 13. In order to provide the noted protection from anticipated vagrant exposure to actinic ultraviolet light during label use, the fabrication process has typically included a "downstream" station at which a continuous web of transparent film bearing a UV-absorbent, or UV-blocker, composition is laminated upon the printed, active monomer face of the label substrate web. To ensure thorough coverage of UV-absorbent composition upon active monomer depositions, the overlay film is normally applied coextensive with the substrate web. A subsequent, final operation in the fabrication stream may be utilized to die-cut and remove extraneous adhesive-backed substrate and overlay film, leaving the desired multiplicity of individual TTI labels on the release sheet backing support web. Along with the excess areas of UV-blocker composition remaining on each label surface, the extraneous overlay film material discarded in this final fabrication operation represents substantial waste of this composition which amounts to a significant component of product cost.

A printing or coating station is situated downstream from the point of active diacetylenic monomer composition application and effects deposition of UV-blocker composition directly upon and in substantial register with the active monomer deposition to provide the latter component optimum protection from incident UV radiation. As in the past, additional printing stations may be provided in the fabrication process for application of desired indicia, such as reference color patches, use instructions, and advertising.

The selective location, according to the invention, of UV-blocker composition only over those areas occupied by active monomer provides additional advantages beyond the direct saving in cost of otherwise wasted UV-absorbent composition. Since, in this improvement, the major extent of the label area is devoid of UV-blocker, indicia may be applied to various portions of that area at a later time and at a greater rate and diversity, and thus more economically, by means of UV-cured printing inks.

Further, and of particular significance in expanding the capabilities of the diacetylenic monomer compositions into multiple response indicator systems, portions of a label face outside the UV-blocker protected TTI site may be employed as sites for indicators, including selected diacetylenic monomer compositions, dedicated as responsive to UV-radiation, thereby incorporating into a single label indicators for both time-temperature and time-radiation integrals relevant to the shelf life of an associated product.

### BRIEF DESCRIPTION OF THE DRAWINGS

Some embodiments of the present invention will be described with reference to the accompanying drawing of which:
FIG. 1 depicts in plan view the general conformation and functional response of a time-temperature indicator (TTI) system device of the prior art typically comprising an active diacetylenic monomer indicator composition and a UV-protective overlay film;
FIG. 2 depicts in elevational cross-section, taken at 2-2 in FIG.1(a), the structure of the prior art TTI system device;
FIG. 3 depicts in elevational cross-section, taken at 3-3 in FIG.4, the structure of a TTI system device embodying the present invention;
FIG. 4 depicts in plan view multiple TTI system devices embodying the present invention;
FIG. 5 presents graphic data depicting the UV-protective efficacy of a device embodying the present invention; and
FIG. 6 presents graphic data depicting the comparative UV-protective efficacy of a device embodying the present invention and one of the prior art.

### DESCRIPTION OF EMBODIMENT OF THE INVENTION

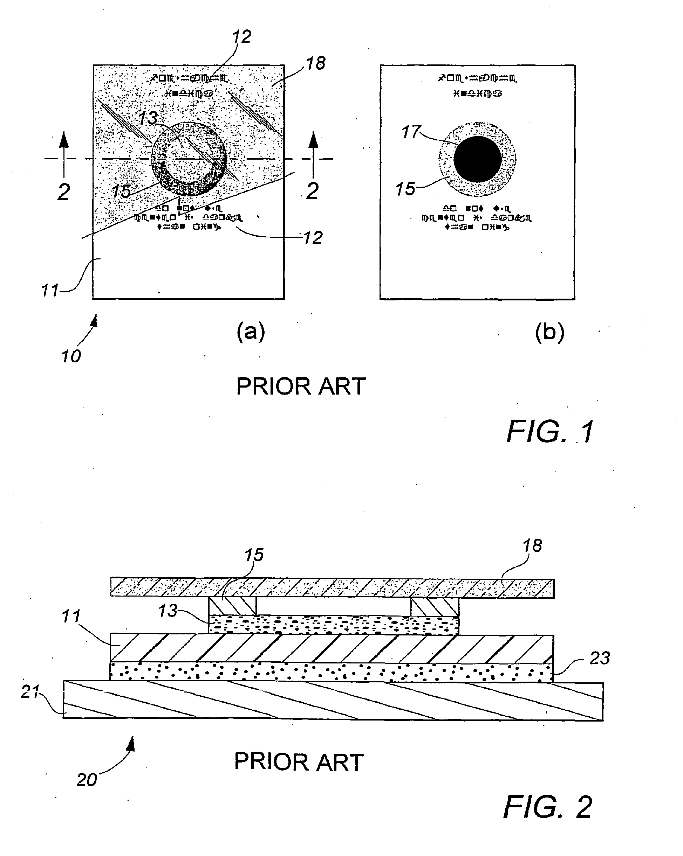

As seen in FIG. 1(a), a TTI system label 10 of the prior art typically comprises a self-adhesive substrate 11 upon which is printed a spot 13 of active diacetylenic monomer indicator composition. A threshold reference color body, such as shown in the form of a ring 15 surrounding spot 13, is printed in close proximity to the active indicator composition and is provided in a color tone closely approximating the color density which will be developed in indicator spot 13 upon accumulation of the time-temperature integral predetermined to be representative of the shelf-life beyond which the intended associated product, e.g., a foodstuff, such as fish or fowl, or a medicament, such as vaccine or medicine, is expected to lose its utility or potency. As an example of such an expired shelf-life, the indicator composition spot 17 of the label in FIG. 1(b) is shown to have polymerized under the influence of ambient temperature beyond the threshold integral period to yield a tone which is far darker than that of reference color ring 15.

The TTI label product may generally bear additional indicia 12 presenting identifying or instructional text and the like. Further, in order to mitigate the effect of incident UV-light on active indicator composition 13, a sheet of transparent film 18, a section only of which is shown in FIG. 1(a), bearing a UV-absorbent blocker composition has often been coextensively laminated to the face of label substrate 11.

FIG. 2 depicts in greater detail a typical construction of the prior art TTI label of FIG. 1. Such a label 20 comprises a release sheet 21 support for label substrate 11 of stable paper or film, e.g., biaxially oriented polypropylene, and its pressure-sensitive self-adhesive coating 23. The selected area, or spot, 13 of active diacetylenic monomer indicator composition is deposited on substrate 11, preferably as an ink or lacquer in a screen, gravure, or other printing operation. A body of reference color, such as a registered ring 15, is printed upon composition 13 to provide a means of ready comparison with polymerization color density level during progression of the target shelf-life. After application of additional desired indicia (not shown), UV-blocking overlay film 18 is laminated, preferably by means of an integral self-adhesive layer (not shown), upon the resulting composite label device.

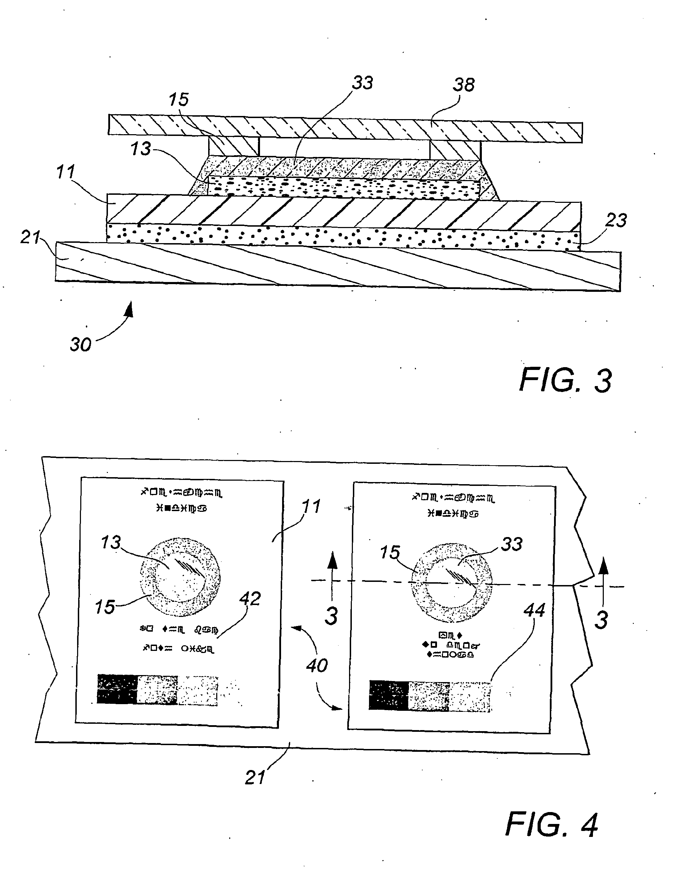

In contrast to the foregoing structure of the prior art, the TTI system label 30 of the present invention is fabricated in a manner to yield the device depicted in FIG. 3. While similar release sheet support web 21 and self-adhesive label substrate 11, 23 materials may be employed to carry the active indicator spot 13 of diacetylenic monomer composition, the present invention departs from past practice by applying a UV-blocking component layer 33 as *a printed UV-absorbent transparent lacquer ink composition* as defined in claim 13, directly over active monomer component 13. The area of applied layer 33 may be in precise register with that of active spot 13; however, the simpler application may preferably be such that layer 33 extends to some degree beyond the area of monomer 13 to thus obviate concern of excessive and costly register control parameters. Such application provides the additional benefit of encompassing the monomer deposition with UV-blocker to minimize lateral exposure to vagrant radiation. In this manner, the UV-sensitive monomer component of the TTI system label is accorded optimum protective coverage with UV-blocker composition 33 while requiring only a minimum amount of such composition and avoiding in great measure the previously encountered costly waste.

A color contrast comparison reference component 15 may be printed in turn with other desired indicia according to prior practice, but with the advantageous exception that improved and highly preferred UV-setting compositions and inks may now be employed without concern of deleteriously affecting the active monomer indicator composition with processing UV light. The utility of reference component ring 15 remains as with prior TTI labels in that, as seen in FIG. 4, the color density change progression of indicator 13 may be observed at any time through visible-transparent UV-blocker film 33 revealed at the annular opening in reference ring 15. As an optional component to provide protection from physical abrasion or abuse, substantially inert, transparent film 38 may be applied over the label product in the manner previously employed with a UV-blocker overlay film 18.

In addition to a capability for subsequent application of printed indicia 42, such as point-of-sale information, in label areas now unobstructed by UV-blocker, a further embodiment afforded by the invention may be seen in FIG. 4 which generally depicts a multiplicity of TTI system labels 40 as typically arranged in series during continuous printed fabrication on a section of carrier web 21. Areas of label substrate 11 not otherwise occupied by active TTI components, and being devoid of UV-blocking composition, are now available to support active components of a responsive system, represented generally at 44, for indicating an integral of time and UV-light exposure, that is, one constituting an indicator of shelf life *vis-à-vis* ultraviolet light to which an associated foodstuff or other product may, along with excessive heating, be susceptible. The limited and localized disposition of UV-absorbent composition achieved in systems of the present invention has enabled such expanded and complementary indicator utility.

Representative implementations of the present invention may be seen more specifically in the following examples.

### Example I

An active TTI system indicator composition comprising a commonly used substituted diacetylenic monomer is prepared by first ball milling about 9.0 parts by weight of 2,4-hexadiyn-1,6-bis(ethylurea) and 25 parts n-butanol for about 16 hours to obtain a fine particle dispersion which is then mixed with a lacquer solution of about 8.3 parts of ethyl cellulose and 60 parts n-butanol. The mixture is then thinned to a desired printing ink consistency with up to about 85 parts of n-butanol.

A UV-blocker ink composition of printable consistency is prepared by thoroughly mixing about 3.5 parts by weight of 2-2' dihydroxy-4-methoxybenzophenone, 2.5 parts Orosol Yellow dyes, and 2.1 parts ZnO into a lacquer base prepared with about 18.4 parts nitrocellulose, 5.0 parts 2-ethylhexyl-2-cyano-3,3-diphenyl acrylate, and 1 part silicone-based flow adjunct in a solvent mixture of about 45 parts ethyl-3-ethoxy propionate, 5.7 parts isopropanol, 11.4 parts ethyl acetate, and 5.4 parts diacetone alcohol.

The above indicator ink composition is printed as a series of spot images of about 6 mm diameter on a first length of continuous strip substrate web of white-pigmented biaxially oriented polypropylene in a Gallus R250 (Gallus, Inc., Philadelphia, PA) rotary screen printing press utilizing a 39%, 180 µm screen. A second length of substrate web is likewise printed with indicator composition, and at a subsequent press station the buff-colored indicator spotsare overprinted in similar manner with the above UV-blocker ink as substantially registered images of about 8 mm diameter. Samplesare taken from the two printings for comparative testing in the following manner.

Sample sections (A and B)are taken from the first length of printed strip web without UV-blocker overprint. Sample (A) is sealed within an opaque foil envelope for use as a control and sample (B) is retained for use as is. A third sample (C) is taken from the second length of printed web having UV-blocker overprint for use as is. These samples and controlare exposed together to mid-day sunlight (at about 22°C and 25% relative humidity) with measurements of indicator spot color density being taken at regular intervals with a commercial reflection densitometer (X-Rite 404) operating in the cyan mode until a predetermined threshold color density of a sample is observed.

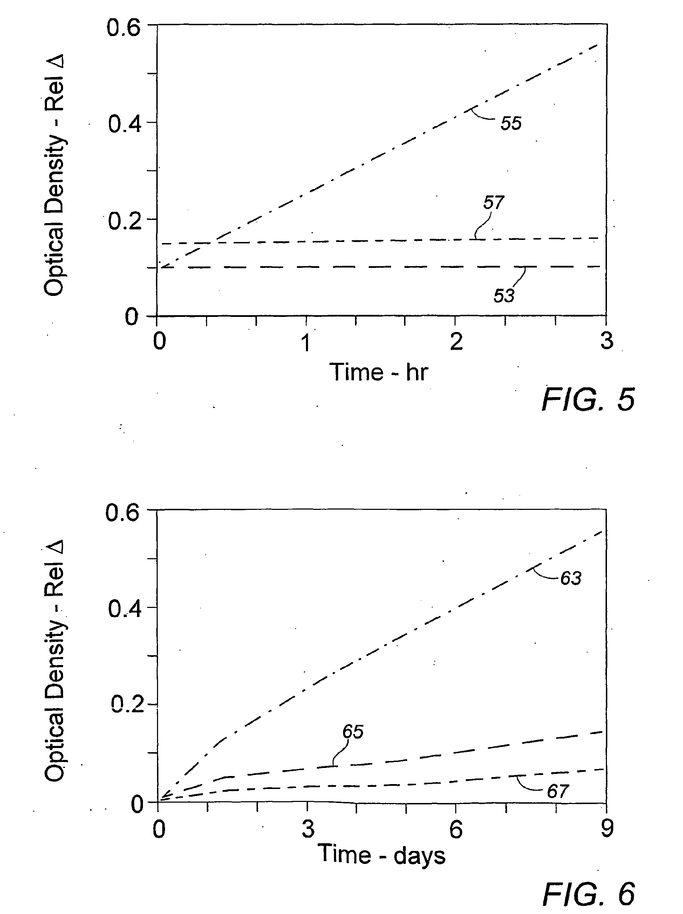

Results of these tests are shown in FIG. 5 as traces of the relative color density change data. As anticipated, data 53 of the enclosed control sample (A) showed no color density change. Data 55 of the unprotected sample (B) indicator spots showed an increase in color density at a regular rate to a final dark blue-black during the nearly 3 hour test period, thus confirming the susceptibility of the active indicator composition to the polymerizing influence, at least in part, of ultraviolet light. Data 57 of indicator sample (C) comprising UV-blocker overprint according to the invention, on the other hand, showed substantially no change in color density during the test period, thereby indicating the high efficacy of the overprint embodiment. The offset of these latter data from those of control sample (A) are attributable to the color initially imparted by UV-blocker composition dyes..

### Example II

The comparative efficacy of the present invention and the prior art with respect to exposure over a more limited wavelength range of ultraviolet light, such as would normally be encountered in artificial lighting of refrigerated foodstuff display counters, is tested in the following manner. Samples (D and E) of plain and UV-blocker overprinted active indicator compositionare prepared in the manner of Example I with the exception of utilizing as the active substituted diacetylenic monomer component a co-crystallized 2:1 mixture of 2,4-hexadiyn-1,6-bis(ethylurea) and 2,4-hexadiyn-1,6-bis(propylurea). A comparative sample (F) according to the prior art is prepared by laminating a commercial UV-blocker film over a portion of sample (D) plain active indicator composition web material.

The prepared samplesare exposed to about 2800 lux under a fluorescent light fixture in a constant ambient of about 4°C to substantially simulate storage in a commercial food market display case. As in Example I, regular measurementsare made of active indicator composition color density and the relative color density change dataare plotted to provide the results shown in FIG. 6. The substantially regular increase in UV-initiated color density in plain sample (D) is represented in the data of trace 63 and is seen to reach a maximum during the 9 day test period. The limited increase in color density of the prior art sample during the same period in overlay-protected sample (F) is shown in the data of trace 65, while trace 67 data from sample (E) shows the lesser rate of UV-initiated color increase achieved by the greater efficacy of the present invention arrangement.

Other TTI system label devices are prepared according to the present invention utilizing various monomer components described in the above-noted patent specifications to achieve a range of time-temperature integral endpoints representative of numerous foodstuff and medicament shelf lives. In each instance, in addition to the readily calculable savings in reduced materials waste, the devices responded with improved efficacy as compared with prior art products.

The UV-blocker ink composition described in Example I is but one example of another aspect of the invention which provides transparent printable "ink" compositions containing a UV blocker which are suitable for printing in one or more desired patterns, for example as spot images, onto a continuous strip substrate, overlaid on spots of indicator ink composition, to protect the indicator from the action of UV light.

For example, as described above with reference to Fig. 3, the UV blocker composition can be overprinted in enlarged areas 33 on indicator spots 13 supported on a suitable substrate 11. Desirably, the inventive printable UV blocker composition is sufficiently clear or transparent to permit a functional color change in the underlying ink spot to be clearly perceived. Desirably also the UV blocker composition of the invention yields a film product which is reasonably or completely free of blooming which may obscure or discolor deposited spots of the composition.

Pursuant to this aspect, the invention provides a transparent, printable UV blocker composition as defined in claim 1.

A further useful optional ingredient is a silicone-based flow agent which may be used to improve wetting of the UV blocker composition on the substrate and indicator composition. Other flow agents, for example fluorine-based flow agents may be employed for this purpose, if desired.

Any suitable film-forming polymer can be employed, including, not only nitrocellulose but also alternatives to nitrocellulose, for example vinyl and acrylic co- and terpolymers, such as copolymers of vinyl acetate with crotonic acid and vinyl tert-butylbenzoate; copolymers of acrylic acid and ethyl acrylate with N-tert-butylacrylamide; copolymers of N-octylacrylamide, methyl methacrylate, hydroxypropyl methacrylateacrylic acid and tert-butylaminoethyl methacrylate copolymer; or a terpolymer of acrylic acid, ethyl acrylate and tert-butyl acrylate.

The volatile carrier can comprise a volatile organic solvent system such as is employed in Example 1 or, as alternatives to the solvent system described therein, any suitable solvent may be employed as the volatile carrier, as will be apparent to those skilled in the art. Suitable solvents systems include individual solvents or mixtures selected from the group consisting of heptane, acetate, amyl acetate, butyl acetate, butyl cellosolve acetate, cellosolve acetate, methul cellosolve acetate, acetone, methy ethyl ketone, methyl isobutyl ketone, butyl cellosolve, cellosolve, methyl cellosolve, ethyl alcohol, ispropyl alcohol, butyl alcohol, toluene, and xylene. Other suitable solvent systems will be apparent to those skilled in the art.

If desired, the UV blocking ink composition may include one or more plasticizers to provide film flexibility and to reduce film shrinkage that may occur on drying. Suitable plasticizers include isopropyl alcohol fatty acid esters, C8 alcohol fatty acid esters, organic succinates, phthalates and adipates, and other plasticizers as known to those skilled in the art.

The various ingredients of the UV blocker compositions can be employed in relative amounts or proportions effective for their intended purposes as may be determined without undue experimentation. Some useful ranges of percentages by weight based upon the total weight of the UV blocker composition include:
a range of from about 0.5 to about 35 percent, or of from about 3 to about 20 percent, or desirably from about 7 to about 15 percent of a combined proportion of the UV blocking agents;
from about 2 to about 40 percent, or of from about 10 to about 30 percent, or desirably from about 15 to about 25 percent of a film forming agent; and
from about 20 to about 95 percent, or of from about 40 to about 85 percent, or desirably from about 60 to about 75 percent of a volatile carrier.

The soluble dye is employed in a range of from about 0.1 to about 10 percent, desirably from about 1 to about 5 percent, for example about 2 to 3 percent may be employed. The flow agent, if employed can be used in conventional proportions for example of from about 0.1 to about 5 percent, e.g about 1 percent.

Useful ranges of organic UV blocker include from about 1 to about 10 percent, or about 2 to about 5 percent of a non-lacquer component and from about 0 to about 12 percent of an optional component incorporated in the lacquer composition. Total organic UV blocker can range from about 2 to about 15 percent or from about 4 to about 10 percent.

One useful range of the inorganic UV blocker is from about 0.5 to about 8 percent or from about 1.5 to about 5 percent The proportion of inorganic to organic UV blocker can usefully be from about 1:10 to about 1:1 or from about 1:5 to about 1:3.

Desirably, the UV blocker composition is completely free of any opaque insoluble pigment particles or is sufficiently free of insoluble pigment particles that the useful transparency of the UV blocker film is not impaired.

It will be understood that, many other UV blocker compositions which can be used to fulfill the objectives of the invention will be, or will become, apparent to those skilled in the art, or can be devised without undue experimentation in light of this disclosure.

The includes methods of making and using UV blocker compositions which follow the teaching of Examples 1 and 2 hereinabove but employ the ingredients and/or proportions described in the immediately preceding paragraphs. In particular the invention includes the print-like application of the described UV blocker compositions as protective overlays on time-temperature indicator spots or patches.

The product shelf life monitoring system can be employed in an desired manner for indicating a useful shelf life or other useful purpose and may be employed in an active manner with bar-coded products. To this end, a transparent shelf life monitoring system according to the invention is overlaid on a bar code label or the like so as to render the bar code initially usable. However, the progressive formation of color of the time-temperature indicator component of the monitoring system can ultimately obscure the bar code, signaling the expiration of the product shelf life. Such a use of a shelf life monitoring system is disclosed in U.S. Patent No. 6,5443,925.

## Claims

1. A product shelf life monitoring system for monitoring the shelf life of an associated product, the system comprising a substrate surface bearing an active indicator ink (13) that is based on a diacetylenic monomer compound and printed within a limited area portion of the substrate surface, the active indicator ink (13) being responsive with a visible change to incident thermal energy and to incident ultraviolet radiation, the associated product being susceptible at least to said thermal energy, the system comprising visibly transparent ultraviolet-radiation-absorbent means disposed over the active indicator ink, the ultraviolet-radiation-absorbent means comprising a layer (33) of a visibly transparent, ultraviolet radiation-absorbent ink covering the active indicator ink (13) and situated only within said limited area portion of the substrate surface whereby the substrate surface comprises an extent outside the limited area portion of the substrate surface that is devoid of the layer (33) of ultraviolet radiation-absorbent ink; said product being **characterized in that** said ultraviolet radiation-absorbent ink includes:
- 2-2' dihydroxy-4-methoxybenzophenone;
- zinc oxide;
- Orasol yellow dye;
- a lacquer base; and
- a volatile liquid carrier.

2. A system according to claim 1 **characterized in that** the extent of the substrate surface outside the limited area portion entirely surrounds the limited area portion.

3. A system according to claim 1 or 2 **characterized by** the extent of the substrate surface outside the limited area portion bearing printed indicia formed of an ultraviolet-cured ink.

4. A system according to claim 3 **characterized in that** the printed indicia include a reference component (15) on the ultraviolet radiation-absorbent layer (33), the reference component being printed with the ultraviolet-curable ink and having an appearance to be compared with the appearance of the indicator composition.

5. A system according to any one of the preceding claims, **characterized in that** the extent of the substrate surface outside the limited area portion of the substrate surface bears a secondary active indicator composition (44) responsive with a visible change to incident ultraviolet radiation.

6. A system according to any one of the preceding claims, **characterized in that** the layer (33) of the ultraviolet radiation-absorbent ink is substantially in registration with the active indicator ink.

7. A system according to any one of the preceding claims, **characterized in that** the layer (33) of ultraviolet radiation-absorbent ink registers with the active indicator ink (13) and encompasses the active indicator ink (13).

8. A system according to any one of the preceding claims, **characterized in that** the substrate comprises an adhesive-coated label and a release sheet providing a backing for the label.

9. A multiplicity of product shelf life monitoring system labels according to claim 8 supported on a continuous carrier web suitable for printing **characterized in that** each label comprises a product shelf life monitoring system according to any of Claims 1 to 8.

10. Use of a product shelf life system according to any one of the foregoing claims for monitoring the shelf life of an associated product, the associated product being selected from the group consisting of foodstuff, medicament, vaccine or medicine.

11. A method of making a product shelf life monitoring system according to claim 1 **characterized by** comprising printing a layer (33) of an ultraviolet radiation-absorbent ink comprising effective quantities of each of
- 2-2' dihydroxy-4-methoxybenzophenone;
- zinc oxide;
- Orasol yellow dye;
- a lacquer base; and
- a liquid carrier
to the limited area portion of the substrate surface to cover the active indicator ink (13) and leaving an extent of the substrate surface outside the limited area portion devoid of the layer (33) of ultraviolet radiation-absorbent ink.

12. A method according to claim 11 **characterized by** comprising printing printed indicia to portions of the shelf life monitoring system outside the indicator area employing an ultraviolet-curable printing ink and applying ultraviolet light to cure the ink.

13. An ultraviolet radiation-absorbent ink composition, comprising:
- 2-2' dihydroxy-4-methoxybenzophenone;
- zinc oxide;
- Orasol yellow dye;
- a lacquer base; and
- a volatile liquid carrier.

## Patentansprüche

1. Produkthaltbarkeitsübenmachungssystem zur Überwachung der Haltbarkeit eines dazugehörigen Produkts, wobei das System eine Substratoberfläche umfaßt, welche eine aktive Indikatortinte (13) trägt, die auf einer diacetylenischen Monomerverbindung basiert und innerhalb eines begrenzten Flächenteils der Substratoberfläche gedruckt ist,
wobei die aktive Indikatortinte (13) mit einer sichtbaren Veränderung auf auftreffende thermische Energie und einfallende Ultraviolettstrahlung reagiert, wobei das dazugehörige Produkt zumindest für die thermische Energie empfänglich bzw. anfällig ist,
wobei das System sichtbare transparente, ultraviolette Strahlung absorbierende Mittel, angeordnet über der aktiven Indikatortinte, umfaßt, wobei das ultraviolette Strahlung absorbierende Mittel eine Schicht (33) einer sichtbaren transparenten, ultraviolette Strahlung absorbierenden Tinte umfaßt, welche die aktive Indikatortinte (13) bedeckt und sich nur innerhalb des begrenzten Flächenteils der Substratoberfläche befindet, wodurch die Substratoberfläche einen Überstand außerhalb des begrenzten Flächenteils der Substratoberfläche umfaßt, welche frei von der Schicht (33) von ultraviolette Strahlung absorbierender Tinte ist,
wobei das Produkt **dadurch gekennzeichnet ist, daß** die ultraviolette Strahlung absorbierende Tinte
- 2,2'-Dihydroxy.4-methoxybenzophenon;
- Zinkoxid;
- Gelbfarbstoff;
- eine Lackbasis, und
- einen flüchtigen flüssigen Träger
einschließt.

2. System nach Anspruch 1, **dadurch gekennzeichnet, daß** der Überstand der Substratoberfläche außerhalb des begrenzten Flächenteils vollständig den begrenzten Flächenteil umgibt.

3. System nach Anspruch 1 oder 2, **dadurch gekennzeichnet, daß** der Überstand der Substratoberfläche außerhalb des begrenzten Flächenteils gedruckte Angaben trägt, gebildet aus einer ultraviolett-gehärteten Tinte.

4. System nach Anspruch 3, **dadurch gekennzeichnet, daß** die gedruckten Angaben eine Referenzkomponente (15) auf der ultraviolette Strahlung absorbierenden Schicht (33) einschließen, wobei die Referenzkomponente mit der ultraviolett-härtbaren Tinte gedruckt ist und eine Erscheinung aufweist, welche mit der Erscheinung der Indikatorzusammensetzung zu vergleichen ist.

5. System nach einem der vorhergehenden Ansprüche, **dadurch gekennzeichnet, daß** der Überstand der Substratoberfläche außerhalb des begrenzten Flächenteils der Substratoberfläche eine sekuridäre aktive Indikatorzusammensetzung (44) trägt, welche mit einer sichtbaren Veränderung auf einfallende Ultraviolettstrahlung reagiert.

6. System nach einem der vorhergehenden Ansprüche, **dadurch gekennzeichnet, daß** die Schicht (33) der ultraviolette Strahlung absorbierende Tinte im wesentlichen in Deckung mit der aktiven Indikatortinte ist.

7. System nach einem der vorhergehenden Ansprüche, **dadurch gekennzeichnet,**
. **daß** sich die Schicht (33) der ultraviolette Strahlung absorbierende Tinte mit der aktiven Indikatortinte (13) deckt und die aktive Indikatortinte (13) einschließt.

8. System nach einem der vorhergehenden Ansprüche, **dadurch gekennzeichnet, daß** das Substrat einen mit Haftmittel beschichteten Aufkleber und ein Ablöseblatt, welches einen Träger für den Aufkleber bereitstellt, umfaßt.

9. Eine Vielzahl von Produkthaftbarkeftsüberwachungssystemaufklebern nach Anspruch 8, gestützt auf einem kontinuierlichen Trägergewebe, welches zum Bedrucken geeignet ist, **dadurch gekennzeichnet, daß** jeder Aufkleber ein Produkthattbarkeitsüberwachungssystem nach einem der Ansprüche 1 bis 8 umfaßt.

10. Verwendung eines Produkthaltbarkeitssystems nach einem der vorhergehenden Anspruche zur Überwachung der Haltbarkeit eines dazugehörigen Produkts, wobei das dazugehörige Produkt ausgewählt ist aus der Gruppe, bestehend aus Nahrungsmitteln, Medikamenten, Impfstoffen oder Medizin.

11. Verfahren zum Herstellen eines Produkthattbarkeitsüberwachungssystems nach Anspruch 1, **dadurch gekennzeichnet, daß** es das Drucken einer Schicht (33) einer ultraviolette Strahlung absorbierenden Tinte, umfassend wirksame Mengen von jeweils
- 2,2'-Dihydroxy-4-methoxybenzophenon;
- Zinkoxid:
- Orasol Gelbfarbstoff;
- einer Lackbasis;
- und eines flüssigen Trägers
auf den begrenzten Flächenteil der Substratoberfläche, um die aktive Indikatortinte (13) und zu bedecken,
und das Freilassen eines Überstands der Substratoberfläche außerhalb des begrenzten Flächenteils von der Schicht (33) ultraviolette Strahlung absorbierender Tinte, umfaßt.

12. Verfahren nach anspruch 11, **dadurch gekennzeichnet, daß** es das Drucken gedruckter Angaben auf Teile des Haltbarkeitsüberwachungssystems außerhalb des Indikatorbereichs unter Verwendung einer ultraviolett härtbaren Drucktinte und
das Zuführen ultravioletten Lichts, um die Tinte zu erhärten, umfaßt.

13. Ultraviolette Strahlung absorbierende Tintenzusammensetzung, umfassend:
- 2,2'-Dihydroxy-4-methoxybenzophenon;
- Zinkoxid;
- Orasol Gelbfarbstoff
- eine und
- einen flüchtigen flüssigen Träger.

## Revendications

1. Système de gestion de la durée de conservation de produits pour la gestion de la durée de conservation d'un produit associé, le système comprenant une surface de substrat portant une encre indicatrice active (13) qui est basée sur un composé de monomère di-acétylénique et imprimée dans une partie limitée de la surface du substrat, l'encre indicatrice active (13) étant sensible à un changement visible à l'énergie thermique incidente et au rayonnement ultraviolet incident, le produit associé étant sensible au moins à ladite énergie thermique, le système comprenant un moyen d'absorption de rayonnement ultraviolet visiblement transparent disposé sur l'encre indicatrice active, le moyen d'absorption de rayonnement ultraviolet comprenant une couche (33) d'une encre absorbant le rayonnement ultraviolet visiblement transparente recouvrant l'encre indicatrice active (13) et située seulement dans ladite partie limitée de la surface du substrat grâce à quoi la surface du substrat comprend une étendue à l'extérieur de la partie limitée de la surface du substrat qui est dépourvue de la couche (33) d'encre absorbant le rayonnement ultraviolet ; ledit produit étant **caractérisé en ce que** ladite encre absorbant le rayonnement ultraviolet comporte :
le 2-2'dihydroxy-4-méthoxybenzophénone ;
l'oxyde de zinc ;
le colorant jaune Orasol ;
une base de laque ; et
un support liquide volatil.

2. Système selon la revendication 1, **caractérisé en ce que** l'étendue de la surface du substrat à l'extérieur de la partie limitée entoure entièrement la partie limitée.

3. Système selon la revendication 1 ou 2, **caractérisé par le fait que** l'étendue de la surface du substrat à l'extérieur de la partie limitée porte des signes imprimés constitués d'une encre durcie aux ultraviolets.

4. Système selon la revendication 3, **caractérisé en ce que** les signes imprimés comportent un composant de référence (15) sur la couche (33) absorbant le rayonnement ultraviolet, le composant de référence étant imprimé avec l'encre durcissable aux ultraviolets et ayant un aspect à comparer avec l'aspect de la composition indicatrice.

5. Système selon l'une quelconque des revendications précédentes, **caractérisé en ce que** l'étendue de la surface du substrat à l'extérieur de la partie limitée de la surface du substrat porte une composition indicatrice active secondaire (44) en réponse à un changement visible au rayonnement ultraviolet incident.

6. Système selon l'une quelconque des revendications précédentes, **caractérisé en ce que** la couche (33) de l'encre absorbant le rayonnement ultraviolet est essentiellement en coïncidence avec l'encre indicatrice active.

7. Système selon l'une quelconque des revendications précédentes, **caractérisé en ce que** la couche (33) d'encre absorbant le rayonnement ultraviolet coïncide avec l'encre indicatrice active (13) et recouvre l'encre indicatrice active (13).

8. Système selon l'une quelconque des revendications précédentes, **caractérisé en ce que** le substrat comprend une étiquette revêtue d'un adhésif et une feuille détachable fournissant un support pour l'étiquette.

9. Multiplicité d'étiquettes de système de gestion de la durée de conservation de produits selon la revendication 8 supportées sur une bande de support continue adaptée à l'impression, **caractérisée en ce que** chaque étiquette comprend un système de gestion de la durée de conservation de produits selon l'une des revendications 1 à 8.

10. Utilisation d'un système de gestion de la durée de conservation de produits selon l'une quelconque des revendications précédentes pour la gestion de la durée de conservation d'un produit associé, le produit associé étant choisi dans le groupe constitué de produits alimentaires, de médicaments, de vaccins ou de remèdes.

11. Procédé de fabrication d'un système de gestion de la durée de conservation de produits selon la revendication 1, **caractérisé par** le fait de comprendre le fait d'imprimer une couche (33) d'une encre absorbant le rayonnement ultraviolet comprenant des quantités efficaces de chacun
du 2-2'dihydroxy-4-méthoxybenzophénone ;
de l'oxyde de zinc ;
du colorant jaune Orasol ;
d'une base de laque ; et
d'un support liquide.
à la partie limitée de la surface du substrat pour recouvrir l'encre indicatrice active (13) et de laisser une étendue de la surface du substrat à l'extérieur de la partie limitée dépourvue de la couche (33) d'encre absorbant le rayonnement ultraviolet.

12. Procédé selon la revendication 11, **caractérisé par** le fait de comprendre l'impression de signes imprimés à des parties du système de gestion de la durée de conservation de produits à l'extérieur de la zone indicatrice en utilisant une encre d'impression durcissable aux ultraviolets et l'application d'une lumière ultraviolette pour durcir l'encre.

13. Composition d'encre absorbant le rayonnement ultraviolet, comprenant :
le 2-2'dihydroxy-4-méthoxybenzophénone ;
l'oxyde de zinc ;
le colorant jaune Orasol ;
une base de laque ; et
un support liquide volatil.
